# EUROPÄISCHE PATENTSCHRIFT

(11) **EP 3 716 926 B1**
(45) Veröffentlichungstag und Bekanntmachung des Hinweises auf die Patenterteilung: **04.10.2023**
(21) Anmeldenummer: 18812102.4
(22) Anmeldetag: 26.11.2018
(51) Int. Cl.: A61F 13/08, A61F 2/66, A61F 13/00

(54) **VERFAHREN UND SPANNMITTEL ZUR HERSTELLUNG EINES MEDIZINISCHEN UND/ODER THERAPEUTISCHEN STRUMPFES**
METHOD AND STRETCHING MEANS FOR THE MANUFACTURE OF A MEDICAL AND/OR THERAPEUTICAL STOCKING
PROCÉDÉ ET MOYEN DE TENSION POUR LA FABRICATION D'UN BAS MÉDICAL ET/OU THÉRAPEUTIQUE

(30) Priorität: 27.11.2017 DE 102017127940
(43) Veröffentlichungstag der Anmeldung: 07.10.2020
(73) Patentinhaber: FXF GmbH, 92224 Amberg (DE)
(72) Erfinder: FISCHER, Franz, 92224 Amberg (DE)
(74) Vertreter: Benninger, Johannes
(86) Internationale Anmeldenummer: PCT/EP2018/082493
(87) Internationale Veröffentlichungsnummer: WO 2019/101983

(56) Entgegenhaltungen:
- WO-A2-2007/085216
- DE-A1-102012 017 722
- DE-U1-202017 106 068
- US-A- 808 296
- US-A1- 2012 116 282
- US-A1- 2016 175 159

## Beschreibung

Die vorliegende Erfindung betrifft ein Verfahren zur Herstellung eines medizinischen und/oder therapeutischen Strumpfes gemäß den Merkmalen des unabhängigen Anspruchs 1 und ein Spannmittel zur Durchführung des Verfahrens gemäß den Merkmalen des unabhängigen Anspruchs 12.

Das Textil bzw. das Gestricke oder das Material eines Strumpfes ist im ungetragenen Zustand in der Regel weitgehend entspannt. Beim Tragen eines Strumpfes durch einen menschlichen Fuß wird das Textil bzw. das Gestricke oder das Strumpfmaterial entsprechend der jeweiligen Fußform vorgespannt und geformt. Da der Strumpf, insbesondere das Strumpfmaterial, im ungetragenen bzw. im entspannten Zustand eine andere Form bzw. Spannung als bei einem getragenen bzw. vorgespannten Strumpf aufweist, ist das definierte Aufbringen und/oder das definierte Applizieren von elastischen Zugmitteln auf bestimmten Bereichen des Strumpfmaterials problematisch und schwierig.

Durch die DE 20 2009 004 214 U1 ist eine Anlage zum Erzeugen einer Beschichtung auf textilen Formteilen bekannt, bei welcher eine Form zur Aufnahme des zu beschichtenden Strumpfes vorgesehen ist. Die Form steht mit einer Schwenkeinrichtung in Verbindung, so dass der aufgenommene Strumpf über eine horizontale Achse schwenkbar angeordnet ist. Zum Beschichten des Strumpfes kann dieser zumindest abschnittsweise in ein Tauchbad gesenkt werden.

Die im Stand der Technik gezeigte Lösung hat sich jedoch als nachteilig erwiesen, da durch das Eintauchen des Strumpfes in ein Tauchbad lediglich große Flächen und keine definierten elastischen Abschnitte bzw. Bereiche auf das Strumpfmaterial eines Strumpfes aufgebracht werden können.

Die Gebrauchsmusterschrift DE 20 2017 106 068 U1 beschreibt einen medizinischen und/oder eine Fußstellung beeinflussenden und/oder korrigierenden Strumpf mit einem elastischen Zugbereich, der unter Ausbildung einer zugkrafttragenden Verbindung mit dem Strumpfmaterial in das Strumpfmaterial eingearbeitet ist.

Die Offenlegungsschrift US 808 296 A offenbart einen künstlichen menschlichen Fuß, der in Abschnitten elastische Eigenschaften aufweisen kann.

Der Erfindung liegt die Aufgabe zugrunde, ein Verfahren zur Herstellung eines medizinischen und/oder therapeutischen Strumpfes zur Verfügung zu stellen, mittels welchem elastische Zugmittel auf einfache Art und Weise auf das Strumpfmaterial appliziert und/oder definiert aufgebracht werden können, so dass mittels der jeweiligen Zugmittel Fußstellungen und/oder Fußfehlstellungen beim Tragen des Strumpfes durch einen menschlichen Fuß behandelt und/oder korrigiert werden können. Eine weitere Aufgabe vorliegender Erfindung kann darin gesehen werden, ein Spannmittel zur Verfügung zu stellen, wodurch der Strumpf möglichst einfach vorgespannt werden kann und/oder das Verfahren zur Herstellung eines medizinischen und/oder therapeutischen Strumpfes auf einfache Art und Weise durchgeführt werden kann. Eine weitere Aufgabe vorliegender Erfindung kann darin gesehen werden, einen medizinischen und/oder therapeutischen Strumpf zur Verfügung zu stellen, welcher zur Behandlung und/oder zur Korrektur von Fußstellungen und/oder Fußfehlstellungen oder dergleichen eingesetzt werden kann.

Diese Aufgaben werden durch ein Verfahren zur Herstellung eines medizinischen und/oder therapeutischen Strumpfes mit den Merkmalen im Anspruch 1 und zumindest ein Spannmittel zur Durchführung des Verfahrens mit den Merkmalen im Anspruch 12 gelöst. Weitere vorteilhafte Ausgestaltungen und Weiterbildungen der Erfindung sind in den jeweils abhängigen Ansprüchen angegeben.

Das erfindungsgemäße Verfahren umfasst ein Verfahren zur Herstellung eines medizinischen und/oder therapeutischen Strumpfes. Wenn nachfolgend von einem medizinischen und/oder therapeutischen Strumpf die Rede ist, soll generell auch ein Strumpf umfasst sein, welcher eine Fußstellung und/oder eine Fußfehlstellung korrigieren und/oder beeinflussen kann. Das Verfahren zur Herstellung eines medizinischen und/oder therapeutischen Strumpfes umfasst zumindest die nachfolgenden Schritte:
In einem ersten Schritt wird ein Strumpf mit einem zumindest weitgehend geschlossenen vorderen Bereich und einen damit integral verbundenen Rumpfbereich und Fersenbereich bereitgestellt. Bei dem geschlossenen vorderen Bereich kann es sich beispielsweise um eine gemeinsame Kammer für die Zehen eines menschlichen Fußes handeln. Wahlweise kann der Strumpf im vorderen Bereich zur Aufnahme der Zehen jeweils eine unterschiedliche Anzahl an Kammern umfassen. Es wäre beispielsweise denkbar, dass der Strumpf zwei Kammern umfasst, wobei eine erste Kammer zur Aufnahme einer Großzehe und eine zweite Kammer zur Aufnahme der weiteren Zehen eines menschlichen Fußes vorgesehen sind. Wahlweise kann der Strumpf drei Kammern, vier Kammer oder fünf Kammern umfassen, wobei je nach Anzahl an Kammern verschieden große Aufnahmen für die jeweiligen Zehen bereitgestellt werden. An dieser Stelle soll darauf hingewiesen werden, dass sich grundsätzlich jeder herkömmliche Strumpf zur Herstellung des medizinischen und/oder therapeutischen Strumpfes eignen kann.

In einem weiteren Schritt wird der gesamte Strumpf und/oder mindestens ein definierter Abschnitt des Strumpfes durch Beaufschlagen des Strumpfes und/oder des wenigstens einen definierten Abschnittes mit einer Vorspannkraft, insbesondere durch Einsetzen zumindest eines Spannmittels in den Strumpf, vorgespannt. Das zumindest eine Spannmittel kann eine zwei- oder dreidimensionale, vorzugsweise eine dem menschlichen Fuß ähnliche und/oder simulierende Geometrie und/oder Form aufweisen. Im vorliegenden Zusammenhang wird das Spannmittel teilweise auch als Schablone bezeichnet, was als synonyme Bezeichnung aufgefasst werden kann.

Durch das Einsetzen des zumindest einen Spannmittels bzw. der Schablone kann das Tragen eines Strumpfes durch einen menschlichen Fuß imitiert und/oder simuliert werden, da das Strumpfmaterial wie beim Tragen des Strumpfes durch einen menschlichen Fuß ebenfalls gespannt und/oder vorgespannt wird. Durch das Einsetzen des zumindest einen Spannmittels in den Strumpf können beim Verfahren zur Herstellung eines medizinischen und/oder therapeutischen Strumpfes zumindest näherungsweise realistische und/oder realitätsnahe Bedingungen geschaffen werden. An dieser Stelle wird darauf hingewiesen, dass durch das zumindest eine Spannmittel zumindest näherungsweise die gleichen Bereiche und/oder Partien gebildet werden, wie ein menschlicher Fuß aufweist. Dementsprechend kann ein vorgespannter Strumpf ebenfalls einen Sohlenbereich, einen Fersenbereich, einen Knöchelbereich, einen vorderen Knöchelbereich, einen Achillessehnenbereich oder sonstige weitere Bereiche wie bei einem menschlichen Fuß umfassen, wobei diese Bereiche des menschlichen Fußes zumindest angenähert und näherungsweise bestimmt werden können.

Allerdings kann die Schablonenkontur des Spannmittels eine Kontur vorgeben, die nicht der Fuß- und Beinform eines entspannt stehenden Menschen entspricht, sondern mehr oder weniger von dieser abweicht, was für das gezielte Erzeugen einer Vorspannkraft oder von Vorspannkräften durch die im folgenden Schritt aufgebrachten Zugmittel sinnvoll und vorgegeben sein kann.

In einem nächsten Schritt erfolgt ein definiertes Auflegen und/oder Anordnen und/oder Anheften von mindestens einem elastischen Zugmittel auf das Strumpfmaterial des vorgespannten Strumpfes und/oder des wenigstens einen Abschnittes des Strumpfes. Das mindestens eine auf das Strumpfmaterial aufgelegte und/oder angeordnete elastische Zugmittel kann zusätzlich zumindest ortsfest am Strumpfmaterial fixiert werden, so dass dieses während der weiteren Handhabung und/oder des weiteren Verfahrens nicht verrutschen kann. Hierzu können beispielsweise Befestigungsmittel, wie beispielsweise Nadeln oder dergleichen vorgesehen sein, die zur vorläufigen Fixierung des mindestens einen elastischen Zugmittels am Strumpfmaterial dienen.

Zeitlich vor dem definierten Auflegen und/oder Anordnen und/oder Anheften des mindestens einen elastischen Zugmittels auf das Strumpfmaterial des vorgespannten Strumpfes können zusätzlich Markierungen auf dem Strumpfmaterial angebracht werden, um die Lage des mindestens einen elastischen Zugmittels zu kennzeichnen und das Anordnen bzw. das Auflegen und/oder das Anheften des mindestens einen elastischen Zugmittels zu erleichtern.

Bereits zeitlich vor dem Vorspannen des Strumpfes oder währenddessen kann festgelegt und/oder definiert werden, bei welcher Seite des Strumpfes es sich um die Strumpfinnenseite bzw. der späteren Fußinnenseite und bei welcher Seite des vorgespannten Strumpfes es sich um die Strumpfaußenseite bzw. der späteren Fußaußenseite beim Tragen des Strumpfes durch einen menschlichen Fuß handelt. Das Festlegen und/oder Definieren der Strumpfseiten bzw. der späteren Fußseiten kann insbesondere für das definierte Auflegen und/oder Anordnen des mindestens einen elastischen Zugmittels erforderlich sein. Dies kann insofern erforderlich sein, da unter Berücksichtigung der späteren Fußinnenseite bzw. Fußaußenseite das mindestens eine elastische Zugmittel jeweils entsprechend am Strumpfmaterial anzuordnen ist, so dass beim Tragen des hergestellten medizinischen und/oder therapeutischen Strumpfes durch einen menschlichen Fuß die elastischen Rückzugskräfte und/oder die elastischen Zugkräfte des mindestens einen elastischen Zugmittels sinnvoll auf den menschlichen Fuß wirken können.

Anschließend erfolgt ein Einwirken auf den vorgespannten Strumpf und/oder auf den wenigstens einen definierten Abschnitt des Strumpfes mit dem mindestens einen auf das Strumpfmaterial aufgelegten und/oder angeordneten elastischen Zugmittel mit definierter Temperatur und/oder mit definiertem Druck und/oder unter Verwendung von Haftmittel, so dass hieraus resultierend ein medizinischer und/oder therapeutischer Strumpf mit mindestens einem applizierten und/oder aufgebrachten elastischen Zugmittel gebildet und/oder hergestellt wird.

Bei dem mindestens einen elastischen Zugmittel kann es sich vorzugsweise um ein elastisches Zugband und/oder ein Tape oder dergleichen handeln, welches gegenüber dem Strumpfmaterial mit höheren elastischen Rückzugskräften ausgebildet ist. Durch das zumindest eine auf dem Strumpf applizierte und/oder aufgebrachte elastische Zugmittel können somit Zugkräfte oder sonstige Kräfte gegenüber dem Strumpfmaterial erzeugt werden, welche beim Tragen des Strumpfes durch einen menschlichen Fuß zur Behandlung und/oder Korrektur von Fußstellungen und/oder Fußfehlstellungen dienen.

Es kann vorgesehen sein, dass durch das Einwirken mit definierter Temperatur das mindestens eine auf das Strumpfmaterial aufgelegte und/oder angeordnete und/oder angeheftete elastische Zugmittel zumindest teilweise aufgeschmolzen wird, ohne dass dabei die Elastizität und/oder die gegenüber dem Strumpfmaterial höheren elastischen Rückzugskräfte des mindestens einen elastischen Zugmittels beeinträchtigt und/oder reduziert werden.

Wahlweise kann zeitlich mit oder zeitlich nach der Temperatureinwirkung der vorgespannte Strumpf mit dem auf das Strumpfmaterial aufgelegten und/oder angeordneten und/oder angehefteten mindestens einen elastischen Zugmittel mit definiertem Druck beaufschlagt und/oder eingewirkt werden.

Es kann weiter vorgesehen sein, dass bei aufgehobener Temperatureinwirkung und/oder der wahlweisen Druckeinwirkung das zumindest teilweise aufgeschmolzene mindestens eine elastische Zugmittel erkaltet und/oder erstarrt, wobei sich das mindestens eine elastische Zugmittel mit dem Strumpfmaterial des Strumpfes verbindet. Das Erkalten und/oder Erstarren des zumindest teilweisen aufgeschmolzenen elastischen Zugmittels kann bei Raumtemperatur erfolgen. Wahlweise und/oder zusätzlich kann Kühlluft zugeführt werden, wodurch das Erkalten und/oder Erstarren des zumindest teilweisen aufgeschmolzenen elastischen Zugmittels beschleunigt werden kann.

Zusätzlich und/oder wahlweise kann Haftmittel, wie beispielsweise Klebstoffe oder dergleichen, zwischen dem Strumpfmaterial und dem mindestens einen elastischen Zugmittel aufgetragen werden, so dass zwischen dem Strumpfmaterial und dem mindestens einen elastischen Zugmittel eine stoffschlüssige Verbindung hergestellt und/oder erzeugt wird.

Weiter kann vorgesehen sein, dass zeitlich nach dem Erkalten und/oder Erstarren des mindestens einen auf dem Strumpfmaterial des Strumpfes applizierten und/oder aufgebrachten Zugmittels das zumindest eine Spannmittel aus dem Strumpf entfernt und/oder entnommen wird, so dass der Strumpf, insbesondere das Strumpfmaterial, in seinen ursprünglichen bzw. entspannten, insbesondere in einen nicht vorgespannten Zustand, überführt wird.

Wahlweise kann bereits unmittelbar nach Temperatureinwirkung das zumindest eine Spannmittel aus dem Strumpf entfernt werden, so dass das Erkalten und/oder Erstarren des zumindest teilweisen aufgeschmolzenen elastischen Zugmittels im entspannten bzw. im nicht vorgespannten Zustand erfolgen kann. Das Erkalten und/oder Erstarren des zumindest teilweisen aufgeschmolzenen elastischen Zugmittels kann beispielsweise bei Raumtemperatur und/oder bei Zuführung von Kühlluft erfolgen, wobei bei Zuführung von Kühlluft das Erkalten und/oder Erstarren des zumindest teilweisen aufgeschmolzenen Zugmittels beschleunigt werden kann.

Es kann weiter kann vorgesehen sein, dass das Einwirken auf den Strumpf mit dem mindestens einen auf das Strumpfmaterial aufgelegten und/oder angeordneten und/oder angehefteten elastischen Zugmittel mit definierter Temperatur und/oder mit definiertem Druck mit einer Zeitdauer von wenigstens fünf Sekunden und höchstens sechzig Sekunden erfolgt, wobei die definierte Zeitdauer der Temperatureinwirkung und/oder der Druckeinwirkung jeweils von der Materialzusammensetzung des mindestens elastischen Zugmittels und/oder des Strumpfes abhängen kann.

Weiter kann vorgesehen sein, dass das Einwirken mit definierter Temperatur auf den Strumpf mit dem mindestens einen auf das Strumpfmaterial aufgelegten und/oder angeordneten und/oder angehefteten elastischen Zugmittel mit einer Temperatur von mindestens 100 Grad Celsius und höchstens 270 Grad Celsius erfolgt, wobei die definierte Temperatur jeweils von der Materialzusammensetzung des mindestens elastischen Zugmittels und/oder des Strumpfes abhängen kann.

Weiter kann das Einwirken mit definiertem Druck auf den Strumpf mit dem mindestens einen auf das Strumpfmaterial aufgelegten und/oder angeordneten und/oder angehefteten elastischen Zugmittel mit einem Druck von wenigstens 0,5 Bar und höchstens fünf Bar erfolgen, wobei der definierte Druck jeweils von der Materialzusammensetzung des mindestens elastischen Zugmittels und/oder des Strumpfes abhängen kann.

Sowohl bei der Zeitdauer der Temperatureinwirkung und/oder der Druckeinwirkung als auch bei der Höhe der Temperatur und/oder des Druckes bzw. des Anpressdruckes ist jeweils zu beachten, dass das mindestens eine elastische Zugmittel lediglich zumindest teilweise aufgeschmolzen wird. Das Einwirken mit einer zu geringen oder einer zu hohen Temperatur und/oder das zu lange oder zu kurze Einwirken mit definierter Temperatur auf das mindestens eine elastische Zugmittel kann die Nachteile mit sich bringen, dass sich das mindestens eine elastische Zugmittel nicht ausreichend und/oder dauerhaft mit dem Strumpfmaterial bzw. dem Strumpf verbinden kann und/oder dass die elastischen Eigenschaften bzw. die elastischen Rückzugskräfte des mindestens einen elastischen Zugmittels negativ beeinträchtigt und/oder reduziert werden.

Je nach Krankheitsbild, Fußstellung und/oder Fußfehlstellung des jeweiligen menschlichen Fußes und/oder je nach gewünschtem Einsatzbereich des Strumpfes, wie beispielsweise zur Supination oder Pronation, kann das mindestens eine elastische Zugmittel unterschiedlich auf das Strumpfmaterial des Strumpfes aufgelegt und/oder angeordnet werden bzw. um den Rumpfbereich, der Sohle, der Ferse oder dergleichen des vorgespannten Strumpfes gelegt werden.

Gemäß einer Weiterbildung des erfindungsgemäßen Verfahrens kann vorgesehen sein, dass das mindestens eine elastische Zugmittel auf einer Seite des Strumpfes oder zu beiden Seiten des vorgespannten Strumpfes zwischen dessen Sohlenbereich und oberhalb der Ferse über den Achillessehnenbereich angeordnet und/oder auf das Strumpfmaterial des vorgespannten Strumpfes aufgelegt und/oder angeheftet wird.

Zusätzlich und/oder wahlweise kann das mindestens eine elastische Zugmittel zumindest abschnittsweise horizontal verlaufend von der Strumpfinnenseite über den Rumpfbereich des vorgespannten Strumpfes in Richtung Strumpfaußenseite angeordnet und/oder auf das Strumpfmaterial des vorgespannten Strumpfes aufgelegt und/oder angeheftet werden.

Zusätzlich und/oder wahlweise kann das mindestens eine elastische Zugmittel zumindest abschnittsweise längsgerichtet und/oder zumindest abschnittsweise schräg verlaufend in den vorderen Bereich des vorgespannten Strumpfes angeordnet und/oder auf das Strumpfmaterial des vorgespannten Strumpfes aufgelegt und/oder angeheftet werden.

Gemäß einer Weiterbildung des erfindungsgemäßen Verfahrens kann das mindestens eine elastische Zugmittel schräg verlaufend über den oberen Spannbereich des vorgespannten Strumpfes zur Strumpfinnenseite und/oder zur Strumpfaußenseite angeordnet und/oder auf das Strumpfmaterial des vorgespannten Strumpfes aufgelegt und/oder angeheftet werden.

Zusätzlich und/oder wahlweise kann das mindestens eine elastische Zugmittel unterhalb oder in etwa auf Höhe eines Knöchelbereich des vorgespannten Strumpfes und in ungefähr horizontaler Richtung am vorderen Knöchelbereich des Strumpfes verlaufend angeordnet und/oder auf das Strumpfmaterial des vorgespannten Strumpfes aufgelegt und/oder angeheftet werden.

Wahlweise kann das mindestens eine elastische Zugmittel schräg verlaufend vom Knöchelbereich auf der Strumpfinnenseite über den oberen Spannbereich zur äußeren Sohlenkante des vorgespannten Strumpfes angeordnet und/oder auf das Strumpfmaterial des vorgespannten Strumpfes aufgelegt und/oder angeheftet werden. Zusätzlich kann das mindestens eine elastische Zugmittel verlaufend von der äußeren Sohlenkante des vorgespannten Strumpfes quer zur Strumpflängsrichtung hinter der Zehenaufnahme entlang der Sohle bis zur Sohleninnenkante und/oder in etwa endend im Bereich der Aufnahme für das Großzehengelenk angeordnet und/oder auf das Strumpfmaterial des vorgespannten Strumpfes aufgelegt und/oder angeheftet werden.

Gemäß einer Weiterbildung des Verfahrens kann das mindestens eine elastische Zugmittel horizontal verlaufend vom Knöchelbereich der Strumpfinnenseite über den vorderen Knöchelbereich des vorgespannten Strumpfes angeordnet und/oder auf das Strumpfmaterial des vorgespannten Strumpfes aufgelegt und/oder angeheftet werden. Insbesondere kann das mindestens eine elastische Zugmittel schräg verlaufend über den Knöchelbereich der Strumpfaußenseite zur Ferse des vorgespannten Strumpfes und/oder von dort wieder nach oben zur Knöchelseite verlaufend angeordnet und/oder auf das Strumpfmaterial des vorgespannten Strumpfes aufgelegt und/oder angeheftet werden.

Wahlweise kann das mindestens eine elastische Zugmittel horizontal verlaufend vom vorderen Knöchelbereich schräg unterhalb des Knöchelbereiches des vorgespannten Strumpfes schräg über den Bereich der Achillessehne des Strumpfes zur äußeren Sohlenkante angeordnet und/oder auf das Strumpfmaterial des vorgespannten Strumpfes aufgelegt und/oder angeheftet werden. Zugleich kann das mindestens eine elastische Zugmittel derart angeordnet und/oder auf das Strumpfmaterial des vorgespannten Strumpfes auflegt und/oder angeheftet werden, so dass dieses sich von der äußeren Sohlenkante des Strumpfes quer zur Strumpflängsrichtung hinter dem Zehenbereich bzw. dem vorderen Bereich bis zur Strumpfinnenkante und/oder etwa endend im Bereich des Großzehengelenks fortsetzt bzw. verläuft.

Gemäß einer Weiterbildung des Verfahrens kann das mindestens eine elastische Zugmittel vom Sohlenbereich des vorgespannten Strumpfes schräg aufwärts über den Bereich bzw. Abschnitt der Achillessehe und/oder oberhalb der Ferse des Strumpfes und jeweils beidseitig zurück verlaufend angeordnet und/oder auf das Strumpfmaterial des vorgespannten Strumpfes aufgelegt und/oder angeheftet werden. Weiter kann das mindestens eine elastische Zugmittel derart angeordnet und/oder auf das Strumpfmaterial des vorgespannten Strumpfes auflegt und/oder angeheftet werden, dass sich dieses im mittleren Sohlenbereich des vorgespannten Strumpfes kreuzt und sich innen über den Spann bzw. Rumpf des vorgespannten Strumpfes fortsetzen kann, vorzugsweise deutlich beabstandet zum Abschnitt des Großzehengelenks und außen in Richtung des Abschnitts der kleinen Zehe gering beabstandet.

Aufgrund der unterschiedlichen Anordnung des mindestens einen elastischen Zugmittels auf dem Strumpfmaterial des Strumpfes können medizinische und/oder therapeutische Strümpfe hergestellt werden, mittels welchen unterschiedliche Fußstellungen und/oder Fußfehlstellungen korrigiert und/oder behandelt werden können sowie welche einen unterschiedlichen Einsatzbereich haben.

Die Erfindung sieht weiter zumindest ein Spannmittel vor, welches zur Durchführung des Verfahrens gemäß einem der Ansprüche 1 bis 11 ausgebildet ist. Das Spannmittel umfasst zumindest näherungsweise Bereiche und/oder Partien, die ein menschlicher Fuß aufweist, wobei das Spannmittel eine Kontur vorgibt, die von der Fuß- und Beinform eines entspannt stehenden Menschen abweicht, wobei das Spannmittel durch eine flache, scheibenartige Schablone gebildet wird.

Das Spannmittel weist eine dem menschlichen Fuß ähnliche und/oder simulierende Geometrie und/oder Form auf, nämlich einen Sohlenbereich, einen Knöchelbereich, einen vorderen Knöchelbereich, einen Achillessehnenbereich und/oder dergleichen. Dementsprechend können mit dem zumindest einen Spannmittel vergleichsweise ähnliche Bereiche und/oder Partien wie bei einem menschlichen Fuß nachgestellt werden, so dass ein durch das zumindest eine Spannmittel vorgespannter Strumpf analog zum menschlichen Fuß ebenfalls einen Sohlenbereich, einen Knöchelbereich, einen vorderen Knöchelbereich, einen Achillessehnenbereich und/oder dergleichen aufweisen kann. Damit kann die Anatomie eines menschlichen Fußes zumindest näherungsweise nachempfunden werden.

Um Faltenbildung beim Auflegen und/oder Anordnen und/oder Anheften des mindestens einen elastischen Zugmittels zu vermeiden, kann das zumindest eine Spannmittel etwa im vorderen Knöchelbereich zumindest eine Ausbuchtung bzw. Erweiterung aufweisen. Die zumindest eine Ausbuchtung bzw. Erweiterung kann vorzugsweise auf Höhe der zumindest einen Einbuchtung bzw. Einkerbung des Sohlenbereichs liegen bzw. gegenüber dieser angeordnet sein.

Weiter kann es vorgesehen sein, dass das zumindest eine Spannmittel in einen Strumpf einsetzbar ist, wobei durch das in den Strumpf eingesetzte zumindest eine Spannmittel eine Vorspannung des Strumpfes bewirkbar und/oder erzeugbar ist. Durch das Einsetzen des zumindest einen Spannmittels kann einem Tragen des Strumpfes durch einen menschlichen Fuß, insbesondere die durch das Tragen des Strumpfes erzeugte bzw. bewirkte Vorspannung bzw. Spannung des Strumpfmaterials zumindest weitgehend nachgestellt werden kann.

Insbesondere kann das zumindest eine Spannmittel im Achillessehnenbereich und im Sohlenbereich zumindest eine Einbuchtung bzw. Einkerbung aufweisen, d.h. das zumindest eine Spannmittel kann in diesen Bereichen eine unterschnittene Form aufweisen. Die zumindest eine Einbuchtung bzw. Einkerbung kann geschwungen und/oder halbkreisförmig und/oder oval ausgebildet sein. Dadurch kann zudem die Distanz zwischen Achillessehnenbereich und Sohlenbereich, insbesondere beim Auflegen und/oder Anordnen und/oder Anheften des mindestens einen elastischen Zugmittels auf das Strumpfmaterial, verkürzt werden, so dass das mindestens eine Zugmittel beim Tragen des hergestellten medizinischen und/oder therapeutischen Strumpfes in diesen Bereichen deutlich stärker auf Spannung kommen kann.

Es kann vorgesehen sein, dass das zumindest eine Spannmittel ein metallisches und/oder ein nichtmetallisches Material und/oder einen Kunststoff und/oder ein Verbundmaterial oder dergleichen umfasst. Weiter kann das zumindest eine Spannmittel hitzebeständig oder dergleichen ausgebildet sein, so dass auch bei höheren Temperaturen die Form bzw. die Geometrie des zumindest einen Spannmittels beibehalten wird. Es kann vorgesehen sein, dass das zumindest eine Spannmittel korrosionsbeständig oder dergleichen ausgebildet ist.

Das zumindest eine Spannmittel kann einteilig oder mehrteilig ausgebildet sein. Bei einer einteiligen Ausführung des zumindest einen Spannmittels kann das zumindest eine Spannmittel unmittelbar in den Strumpf eingesetzt werden. Bei einer mehrteiligen Ausführung des zumindest einen Spannmittels können Verbindungsmittel bzw. Verbindungselemente vorgesehen sein, über welche die einzelnen Teile des zumindest einen Spannmittels form- und/oder kraftschlüssig zusammengesetzt und/oder zusammengefügt werden. Bei den Verbindungsmitteln kann es sich beispielsweise um Steck- und/oder Rastverbindungen und/oder um Schraub- und/oder Nietverbindungen handeln. Wahlweise können die einzelnen Teile des zumindest einen Spannmittels stoffschlüssig zusammengesetzt und/oder zusammengefügt werden. Denkbare Verbindungstechniken wären beispielsweise Schweißen und/oder Löten oder dergleichen. Grundsätzlich können sich auch sämtliche weitere bekannte Verbindungstechniken eignen.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 verdeutlicht einzelne Schritte zur Umsetzung einer Ausführungsform des erfindungsgemäßen Verfahrens.
Fig. 2 zeigt eine Ausführungsform des zumindest einen Spannmittels in einer schematischen Ansicht.
Fig. 3 zeigt eine Seite des Strumpfes mit mindestens einem auf das Strumpfmaterial applizierten und/oder aufgebrachten elastischen Zugmittel in einer schematischen Ansicht, wobei hier der Strumpf durch das in Fig. 2 dargestellte Spannmittel vorgespannt ist.
Fig. 4 zeigt die gegenüberliegende Seite des in Fig. 3 gezeigten Strumpfes in einer schematischen Ansicht, wobei auch hier der Strumpf durch das in Fig. 2 dargestellte Spannmittel vorgespannt ist.
Fig. 5 zeigt zum Vergleich die Strumpfform eines entspannten Strumpfes und die Strumpfform eines durch ein Spannmittel vorgespannten Strumpfes in einer schematischen Ansicht.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie das erfindungsgemäße Verfahren, das erfindungsgemäße Spannmittel und ein durch das erfindungsgemäße Verfahren hergestellter medizinischer und/oder therapeutischer Strumpf ausgestaltet sein können und stellen keine abschließende Begrenzung dar.

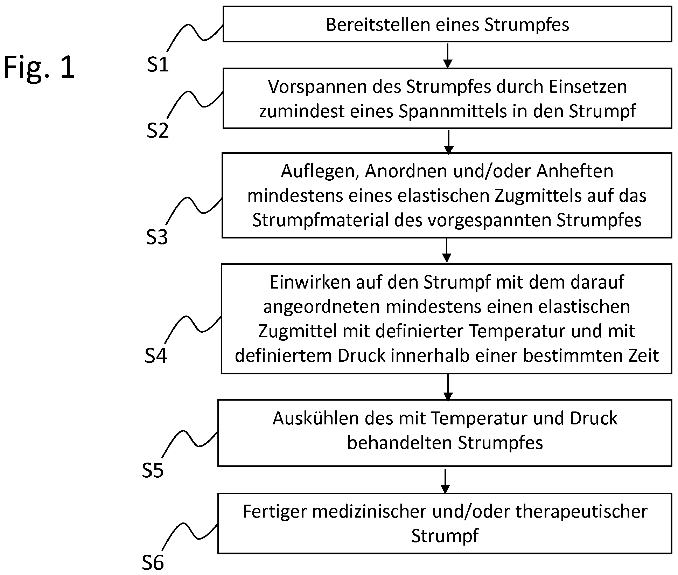

Die Fig. 1 verdeutlicht einzelne, im Zeitablauf aufeinander folgende Schritte zur Umsetzung einer Ausführungsform des Verfahrens zur Herstellung eines medizinischen und/oder therapeutischen Strumpfes 10 in einem Blockdiagramm.

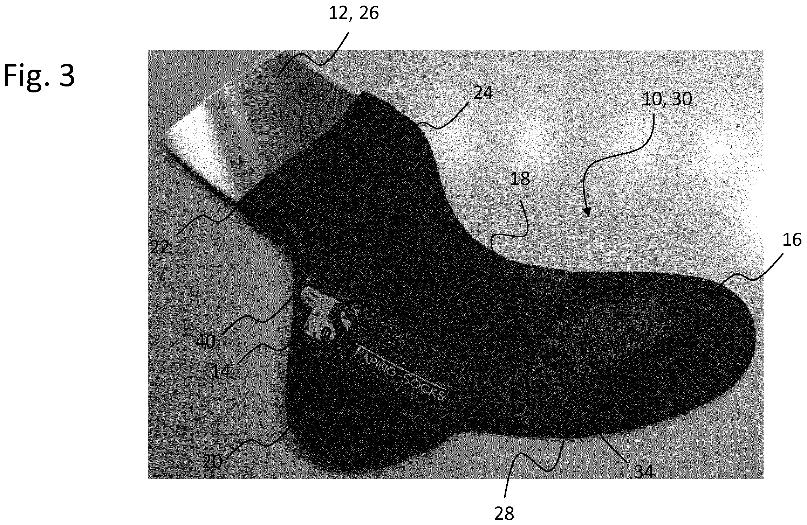

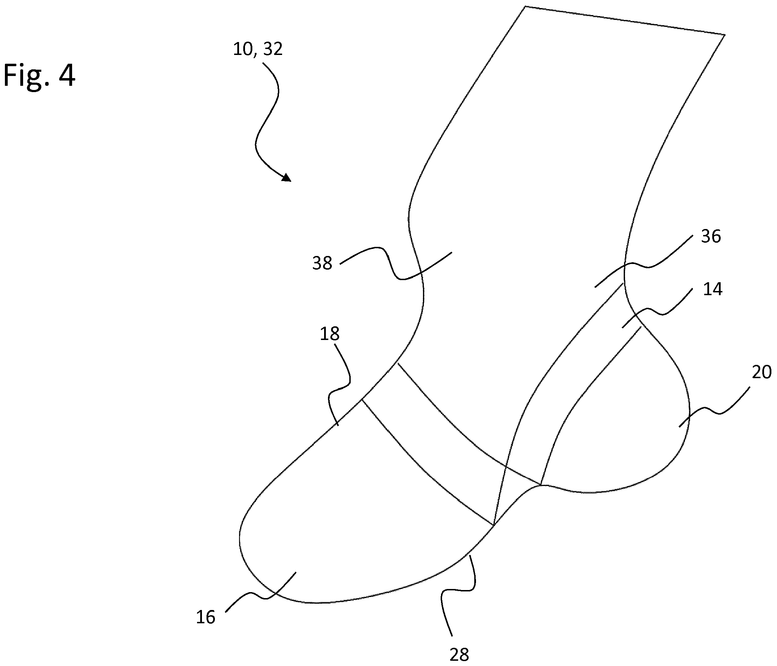

In einem ersten Schritt S1 wird ein Strumpf bereitgestellt, bei dem es sich insbesondere um einen handelsüblichen Strumpf aus einem Stoffgewebe und/oder Stoffgewirke handeln kann. Der Strumpf umfasst einen weitgehend geschlossenen vorderen Bereich 16 und einen damit integral verbundenen Rumpfbereich 18 und Fersenbereich 20 (vgl. Figuren 3 und 4).

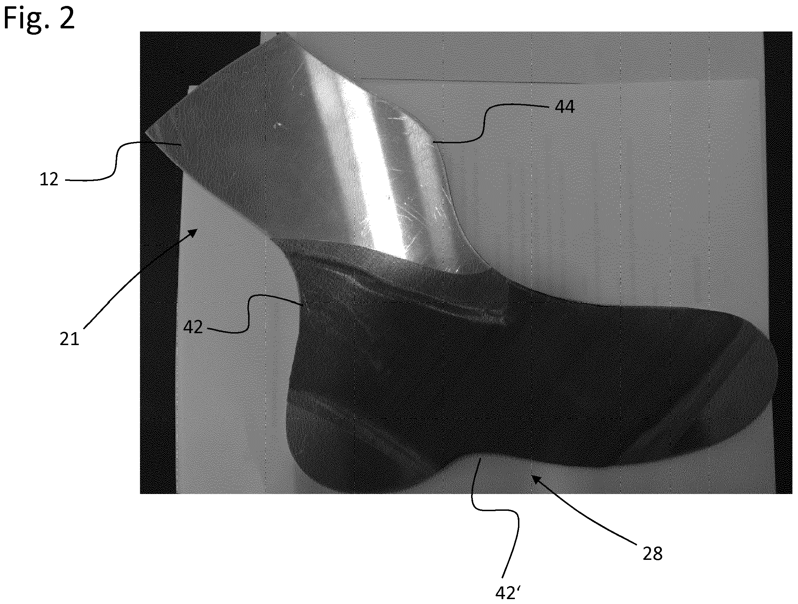

In einem weiteren Schritt S2 wird der bereitgestellte Strumpf vorgespannt, indem zumindest ein Spannmittel 12 (vgl. Fig. 2) in den Strumpf eingesetzt wird, wobei das Spannmittel 12 vollständig oder zumindest abschnittsweise in den Strumpf einsetzbar ist oder eingesetzt wird. Das (zumindest eine) Spannmittel 12 kann vorzugsweise über die obere Öffnung 22 des Strumpfes eingesetzt und bis zum vorderen Bereich 16 bzw. Zehenbereich des Strumpfes geführt werden, so dass der Strumpf bzw. das Strumpfmaterial 24 entsprechend der Kontur des Spannmittels 12 in seiner gesamten Längserstreckung vorgespannt wird. Durch das Einsetzen des Spannmittels 12 in den Strumpf wird das Tragen eines Strumpfes durch einen menschlichen Fuß zumindest angenähert oder ggf. auch weitgehend nachempfunden.

Zugleich wird dadurch festgelegt und/oder definiert, bei welcher Seite des Strumpfes es sich um die Strumpfinnenseite 32 bzw. der späteren Fußinnenseite und bei welcher Seite des vorgespannten Strumpfes es sich um die Strumpfaußenseite 30 bzw. der späteren Fußaußenseite beim Tragen des Strumpfes durch einen menschlichen Fuß handelt.

In Abhängigkeit davon erfolgt im nachfolgenden Schritt S3 ein definiertes Auflegen und/oder Anordnen und/oder Anheften von mindestens einem elastischen Zugmittel 14 auf das Strumpfmaterial 24 des vorgespannten Strumpfes. Da durch das Vorspannen des Strumpfes die Form und die Geometrie eines menschlichen Fußes zumindest weitgehend nachempfunden werden, erfolgt das Auflegen und/oder Anordnen und/oder Anheften des mindestens einen elastischen Zugmittels 14 im Hinblick auf die späteren zu behandelnden Bereiche bzw. Partien eines menschlichen Fußes präziser und genauer als dies etwa bei einem Strumpf ohne eingesetztes Spannmittel der Fall wäre, denn ein solcher Strumpf wäre nicht vorgespannt und wäre daher für das präzise Aufbringen von elastischen Zugmitteln 14 nicht in ausreichendem Maße vorbereitet.

Dementsprechend umfasst ein vorgespannter Strumpf entsprechend den Geometrien und/oder Partien eines menschlichen Fußes ebenfalls einen Sohlenbereich 28, einen Fersenbereich 20, einen Knöchelbereich 36, einen vorderen Knöchelbereich 38, einen Achillessehnenbereich 40 oder sonstige Bereiche eines menschlichen Fußes (vgl. Figuren 3 und 4).

Bei dem mindestens einen elastischen Zugmittel 14 handelt es sich um ein elastisches Zugband und/oder ein Tape, welches gegenüber dem Strumpfmaterial 24 mit höheren elastischen Rückzugskräften als das ebenfalls elastische Strumpfmaterial 24 ausgebildet ist. Durch das mindestens eine elastische Zugmittel 14 werden damit Zugkräfte oder sonstige Kräfte gegenüber dem Strumpfmaterial 24 erzeugt, welche beim Tragen des hergestellten medizinischen und/oder therapeutischen Strumpfes 10 durch einen menschlichen Fuß zur Behandlung und/oder Korrektur von Fußstellungen und/oder Fußfehlstellungen dienen.

Je nach Krankheitsbild eines menschlichen Fußes und/oder gewünschtem Einsatzbereich, wie beispielsweise zur Supination oder Pronation, ist das mindestens eine elastische Zugmittel 14 auf unterschiedliche Art und Weise anzuordnen und/oder auf das Strumpfmaterial 24 aufzulegen. Das präzise und/oder exakte Auflegen des mindestens einen elastischen Zugmittels 14 auf dem Strumpf ist zur Erzeugung von definierten Rückzugskräften und/oder elastischen Zugkräften gegenüber dem Strumpfmaterial 24 hinsichtlich des medizinischen und/oder therapeutischen Behandlungserfolges von wesentlicher Bedeutung.

In einem weiteren Schritt S4 wird auf den vorgespannten Strumpf mit dem mindestens einen auf das Strumpfmaterial 24 aufgelegten und/oder angeordneten und/oder angehefteten Zugmittel 14 mit definierter Temperatur und/oder mit definiertem Druck innerhalb einer vorbestimmten Zeit eingewirkt, so dass das mindestens eine elastische Zugmittel 14 zumindest teilweise aufgeschmolzen wird.

Das Einwirken auf den Strumpf mit dem mindestens einen auf das Strumpfmaterial 24 aufgelegten und/oder angeordneten und/oder angehefteten elastischen Zugmittel 14 mit definierter Temperatur erfolgt mit einer Temperatur von mindestens 100 Grad Celsius und höchstens 270 Grad Celsius. Das Einwirken auf den Strumpf mit dem mindestens einen auf das Strumpfmaterial 24 aufgelegten und/oder angeordneten und/oder angehefteten elastischen Zugmittel 14 mit definiertem Druck erfolgt mit einem Druck von wenigstens 0,5 Bar und höchstens fünf Bar.

Das Einwirken auf den Strumpf mit dem mindestens einen auf das Strumpfmaterial 24 aufgelegten und/oder angeordneten und/oder angehefteten elastischen Zugmittel 14 mit definierter Temperatur und/oder mit definiertem Druck erfolgt mit einer Zeitdauer von wenigstens fünf Sekunden und höchstens sechzig Sekunden.

Sowohl die Höhe der Temperatur und/oder des Druckes als auch die Zeitdauer der Temperatur- und/oder Druckeinwirkung hängen jeweils von der verwendeten Materialzusammensetzung des mindestens einen elastischen Zugmittels 14 und/oder des Strumpfes ab.

Die Kombination aus definierter Temperaturhöhe und/oder der definierten Höhe des Drucks bzw. des Anpressdrucks und definierter Zeitdauer der Temperatureinwirkung trägt dazu bei, dass das mindestens eine elastische Zugmittel 14 zumindest teilweise aufgeschmolzen wird, ohne dass die elastischen Eigenschaften und/oder die gegenüber dem Strumpfmaterial 24 höheren elastischen Rückstellkräfte negativ beeinträchtigt oder reduziert werden.

Zeitlich nach aufgehobener Temperatureinwirkung erstarrt und/oder erkaltet das zumindest teilweise aufgeschmolzene elastische Zugmittel 14 im folgenden Schritt S5, so dass sich das mindestens eine elastische Zugmittel 14 mit dem Strumpfmaterial 24 des Strumpfes verbindet, woraus resultierend ein medizinischer und/oder therapeutischer Strumpf 10 gebildet und/oder hergestellt wird. Das Erkalten und/oder Erstarren und das dadurch bewirkte Verbinden mit dem Strumpfmaterial 24 des zumindest teilweisen aufgeschmolzenen elastischen Zugmittels 14 erfolgt bei Raumtemperatur. Zur Beschleunigung des Abkühlprozesses ist wahlweise Kühlluft zuführbar.

Nachdem das zumindest teilweise aufgeschmolzene elastische Zugmittel 14 erkaltet und/oder erstarrt ist, wird das zumindest eine Spannmittel 12 über die Öffnung 22 des hergestellten medizinischen und/oder therapeutischen Strumpfes 10 entnommen, was durch den folgenden Schritt S6 gekennzeichnet ist. Nach dem Entfernen des zumindest einen Spannmittels 12 nimmt der hergestellte medizinische und/oder therapeutische Strumpf 10 seinen ursprünglichen bzw. entspannten Zustand ein, womit nicht der ursprüngliche Zustand ohne elastische Zugmittel 14, sondern ein neuer ursprünglicher oder entspannter Zustand unter der Einwirkung der elastischen Zugmittel 14 gemeint ist.

Die Fig. 2 zeigt beispielhaft ein Spannmittel 12, welches zur Herstellung eines medizinischen Strumpfes 10 gemäß der anhand der Fig. 1 erläuterten Verfahrensschritte verwendet wird. Das Spannmittel 12 weist eine dem menschlichen Fuß ähnliche und/oder eine die Fußkontur simulierende Form bzw. Geometrie auf und ist einteilig ausgebildet. Das Spannmittel 12 weist in der Regel weiche und/oder geschwungene und/oder abgerundete Konturen und/oder Kanten auf, um das Strumpfmaterial 24 eines Strumpfes vor Beschädigungen zu schützen, zumal auch der Strumpf selbst keine scharfen Kanten oder Ecken in seiner Kontur aufweist.

Das (zumindest eine) Spannmittel 12 kann bspw. durch ein metallisches Material gebildet sein, welches vorzugsweise hitze- und/oder korrosionsbeständig ausgebildet ist. Ebenso denkbar sind jedoch auch andere hitzebeständige und formstabile Materialien wie Holz, Kunststoff oder Verbundwerkstoffe.

Aufgrund einer dem menschlichen Fuß ähnlichen und/oder simulierenden Geometrie bzw. Form des zumindest einen Spannmittels 12 sind damit zumindest näherungsweise die gleichen Bereiche und/oder Partien wie bei einem menschlichen Fuß darstellbar. Dementsprechend umfasst ein durch das zumindest eine Spannmittel 12 vorgespannter Strumpf analog zu den Geometrien und/oder Partien eines menschlichen Fußes ebenfalls einen Sohlenbereich 28, einen Fersenbereich 20, einen Knöchelbereich 36, einen vorderen Knöchelbereich 38, einen Achillessehnenbereich 40 oder sonstige Bereiche eines menschlichen Fußes (vgl. Figuren 3 und 4).

Insbesondere weist das zumindest eine Spannmittel 12 im Achillessehnenbereich 40 und im Sohlenbereich 28 zumindest eine Einbuchtung bzw. Einkerbung 42, 42` auf, welche eine geschwungene und/oder ovale Form aufweist, d.h. in diesen Bereichen weist das zumindest eine Spannmittel 12 eine unterschnittene Form auf. Dadurch wird die Distanz zwischen Achillessehnenbereich 40 und Sohlenbereich 28, insbesondere beim Auflegen und/oder Anordnen und/oder Anheften des mindestens einen elastischen Zugmittels 14 auf das Strumpfmaterial 24, verkürzt, so dass das mindestens eine Zugmittel 14 beim Tragen des hergestellten medizinischen und/oder therapeutischen Strumpfes 10 in diesen Bereichen deutlich stärker auf Spannung kommt (vgl. Figuren 3 und 4). Um Faltenbildung beim Auflegen und/oder Anordnen und/oder Anheften des mindestens einen elastischen Zugmittels 14 zu vermeiden, weist das zumindest eine Spannmittel 12 etwa im vorderen Knöchelbereich 38 zumindest eine Ausbuchtung bzw. Erweiterung 44 auf. Die zumindest eine Ausbuchtung bzw. Erweiterung 44 liegt vorzugsweise in etwa auf Höhe der im Sohlenbereich 28 vorgesehenen zumindest einen Einbuchtung bzw. Einkerbung 42, 42`.

Es sei an dieser Stelle ausdrücklich klargestellt, dass es sich bei dem Spannmittel 12 um eine Art einer flachen, scheibenartigen Schablone handelt, die in den Strumpf eingeschoben wird, um dem Strumpf eine von der normalerweise vorliegenden schlaffen Gewebe- oder Gewirkestruktur bzw. -kontur abweichende Kontur zu geben, da diese abweichende Kontur zur Definition bestimmter Vorspannungskräfte und -richtungen im Zusammenhang mit der Aufbringung der Zugbänder und/oder elastischen Zugmittel 14 notwendig ist. Die Materialstärke der Schablone oder des Spannmittels 12 kann in einer Größenordnung von mindestens einem Millimeter bis zu einer sinnvollen Stärke von einem Zentimeter oder mehr liegen, wobei die umlaufenden Kanten vorzugsweise abgerundet sind, um nicht den Strumpf zu beschädigen, wenn die Schablone eingeschoben und der Strumpf dabei vorgespannt wird.

Wie es die Fig. 2 erkennen lässt, ist der Sohlenbereich 28 zwischen vorderem Bereich 16 und Fersenbereich 20 deutlich eingezogen, während sowohl der vordere Bereich 16 als auch der Fersenbereich 20 durch ausgeprägte Rundungen deutlich betont sind und den Strumpf entsprechend vorspannen, wenn die Schablone oder das Spannmittel 12 dort eingeschoben ist. Auch ist der Knöchelbereich 21 der Schablone oberhalb des Fersenbereichs 20 und in Richtung des oberen Abschlusses nach hinten geneigt, was einem stark gestreckten Fuß mit abgesenktem Vorderfußbereich entspräche. Alle diese Abweichungen der Schablonenform des Spannmittels 12 von einer entspannten Fußstellung bei aufrecht stehendem Strumpfträger sorgen dafür, dass beim nachfolgenden Aufbringen der elastischen Zugmittel 14 eine definierte Vorspannung des Strumpfes bereits vorhanden ist, so dass nach dem Fixieren der Zugmittel 14 und dem Entfernen der Schablone 12 aus dem Strumpf die gewünschten definierten Vorspannkräfte zur Beeinflussung einer von einer Normalstellung abweichenden Fußstellung beim Tragen des Strumpfes vorhanden sind und ihre Wirkung entfalten können.

Die Figuren 3 und 4 zeigen jeweils einen medizinischen und/oder therapeutischen Strumpf 10 von zwei verschiedenen Seiten. Der Strumpf 10 ist durch das in Fig. 2 gezeigte zumindest eine Spannmittel 12 - d.h. durch die das Spannmittel 12 bildende Schablone - vorgespannt und gemäß dem in Fig. 1 dargestellten Verfahren hergestellt.

Zur Vorspannung des Strumpfes wird die Schablone oder das Spannmittel 12 über die obere Öffnung 22 des Strumpfes (oberhalb des Knöchelbereichs 21; vgl. Fig. 2) eingesetzt, bis das Spannmittel 12 den vorderen Bereich 16 des Strumpfes erreicht. Da das Spannmittel 12 eine größere bzw. eine längere Form als der nicht vorgespannte, aber auch als der vorgespannte Strumpf umfasst, ragt das zumindest eine in den Strumpf eingesetzte Spannmittel 12 zumindest teilweise aus der Öffnung 22 des Strumpfes heraus. Dieser Abschnitt wird auch als herausragender bzw. überstehender Abschnitt 26 des Spannmittels 12 bezeichnet. Umgekehrt gilt, dass das Spannmittel 12 zumindest abschnittsweise in den Strumpf einsetzbar ist.

Durch das Vorspannen des Strumpfes entstehen zwei Seiten des Strumpfes, wobei festgelegt und/oder definiert wird, bei welcher Seite es sich um die Strumpfaußenseite 30 bzw. der späteren Fußaußenseite (vgl. Fig. 3) sowie bei welcher Seite des Strumpfes es sich um die Strumpfinnenseite 32 bzw. der späteren Fußinnenseite (vgl. Fig. 4) handelt. Entsprechend der jeweiligen Seite des Strumpfes ist das mindestens eine elastische Zugmittel 14 jeweils unterschiedlich auf das Strumpfmaterial 24 des vorgespannten Strumpfes aufzulegen bzw. anzuordnen bzw. anzuheften. Bei dem mindestens einen elastischen Zugmittel 14 handelt es sich vorzugsweise um ein elastisches Zugband und/oder ein Tape oder dergleichen (bzw. um mehrere solche Tapes oder Zugbänder), welches gegenüber dem Strumpfmaterial 24 mit höheren elastischen Rückzugskräften ausgebildet ist. Durch das mindestens eine auf dem Strumpf applizierte und/oder aufgebrachte elastische Zugmittel 14 werden somit Zugkräfte oder sonstige Kräfte gegenüber dem Strumpfmaterial 24 erzeugt, welche beim Tragen des Strumpfes durch einen menschlichen Fuß zur Behandlung und/oder Korrektur von Fußstellungen und/oder Fußfehlstellungen dienen.

In den Figuren 3 und 4 wird die Anordnung des mindestens einen auf dem Strumpfmaterial 24 applizierten und/oder aufgebrachten elastischen Zugmittels 14 deutlich. Nach Vorspannung des Strumpfes durch das zumindest eine Spannmittel 12 ist das mindestens eine elastische Zugmittel 14 zu beiden Seiten des Strumpfes zwischen dessen Sohlenbereich 28 und oberhalb der Ferse 20 über den Achillessehnenbereich 40 aufgelegt und/oder angeordnet (vgl. Figuren 3 und 4). Das mindestens eine elastische Zugmittel 14 ist weiter zumindest abschnittsweise horizontal von der Strumpfinnenseite 32 über den Rumpfbereich 18 des Strumpfes in Richtung Strumpfaußenseite 30 aufgelegt und/oder angeordnet (vgl. Fig. 4). Zusätzlich verläuft das mindestens eine elastische Zugmittel 14 zumindest abschnittsweise schräg verlaufend in den vorderen Strumpfbereich 16 des Strumpfes (vgl. Fig. 3).

Das mindestens eine elastische Zugmittel 14, welches zumindest abschnittsweise horizontal von der Strumpfinnenseite 32 über den Rumpfbereich 18 des Strumpfes in Richtung Strumpfaußenseite 30 sowie zumindest abschnittsweise schräg in den vorderen Strumpfbereich 16 des Strumpfes verläuft, umfasst eine Vielzahl an zum Teil nicht dargestellten Aussparungen 34. Die Aussparungen 34 sind entlang der Längserstreckung des mindestens einen elastischen Zugmittels 14 angeordnet und weisen einen rundlichen und/oder ovalen Querschnitt auf und/oder sind schlitzartig ausgebildet.

Das mindestens eine elastische Zugmittel 14 ist gemäß den Figuren 3 und 4 derart angeordnet, dass der medizinische und/oder therapeutische Strumpf 10 zur Pronation eingesetzt wird. Bei einer Pronation kann durch das mindestens eine elastische Zugmittel 14 insbesondere eine Kraftkomponente bzw. eine Zugkraft erzeugt werden, welche beim Tragen des medizinischen und/oder therapeutischen Strumpfs 10 durch einen menschlichen Fuß auf der Fußinnenseite in Richtung des Fußknöchels wirkt. Auf diese Weise kann der Fuß auf seiner Fußinnenseite gegenüber der Fußaußenseite angehoben und gestützt werden, in dem das Fersenbein hoch gehalten wird, so dass eine andere Kipprichtung erzeugt wird.

Wie bereits in Fig. 1 dargestellt, ist zeitlich nach dem definierten Auflegen und/oder Anordnen des mindestens einen elastischen Zugmittels 14 auf das Strumpfmaterial 24 eine Temperatureinwirkung und/oder eine Druckeinwirkung innerhalb einer definierten Zeit erforderlich, so dass das mindestens eine elastische Zugmittel 14 zumindest teilweise aufschmolzen wird (vgl. Fig. 1). Nach aufgehobener Temperatureinwirkung und/oder eine Druckeinwirkung erstarrt und/oder erkaltet das zumindest teilweise aufgeschmolzene elastische Zugmittel 14, so dass sich dieses mit dem Strumpfmaterial 24 des Strumpfes verbindet, wie es in den Figuren 3 und 4 dargestellt ist.

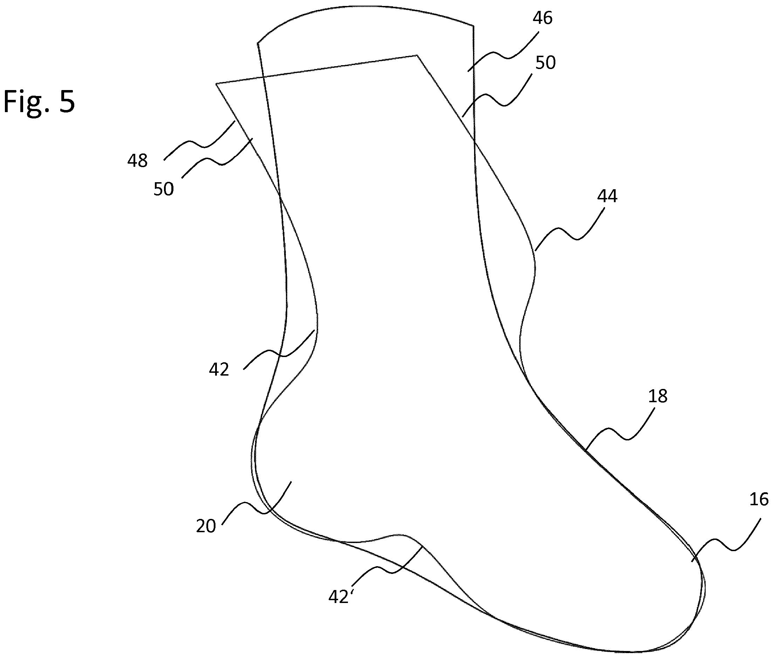

Die Fig. 5 zeigt in einem Vergleich zwei Strumpfformen, wobei die eine Strumpfform den Strumpf in einem entspannten Zustand zeigt und die andere Strumpfform den Strumpf in einem vorgespannten Zustand zeigt, in welchem das in Fig. 2 gezeigte Spannmittel 12 eingesetzt ist. Beide Strumpfformen weisen einen zumindest weitgehend geschlossenen vorderen Bereich 16 und einen damit integral verbundenen Rumpfbereich 18 und einen Fersenbereich 20 auf, wobei sich an den Fersenbereich 20 ein länglicher Schaft 50 anschließt.

Der bereitgestellte Strumpf ist so ausgebildet, dass dieser zunächst eine entspannte Strumpfform 46 aufweist, welche zumindest näherungsweise L-förmig ausgebildet ist.

Das Vorspannen des bereitgestellten Strumpfes durch Einsetzen zumindest eines Spannmittels 12 (vgl. Figuren 2 bis 3) in den Strumpf bewirkt eine zumindest vorübergehende Veränderung der Strumpfform bzw. eine vorgespannte Strumpfform 48 des Strumpfes. Entsprechend der Form und/oder der Geometrie des zumindest einen in den Strumpf eingesetzten Spannmittels 12 (vgl. Fig. 2) weist der Strumpf im Achillessehnenbereich 40 und im Sohlenbereich 28 zumindest abschnittsweise zumindest eine Einbuchtung bzw. Einkerbung 42, 42` auf, welche eine geschwungene und/oder eine ovale Form aufweist. Dadurch wird die Distanz zwischen Achillessehnenbereich 40 und Sohlenbereich 28, insbesondere beim Auflegen und/oder Anordnen und/oder Anheften des mindestens einen elastischen Zugmittels 14 auf das Strumpfmaterial 24, verkürzt, so dass das mindestens eine elastische Zugmittel 14 in diesen Bereichen beim Tragen des hergestellten medizinischen und/oder therapeutischen Strumpfes 10 deutlich stärker auf Spannung kommt. Um Faltenbildung beim Auflegen und/oder Anlegen und/oder Anheften des mindestens einen elastischen Zugmittels 14 zu vermeiden, weist der vorgespannte Strumpf analog zum eingesetzten Spannmittel 12 etwa im vorderen Knöchelbereich 38 zumindest eine Ausbuchtung bzw. Erweiterung 44 auf. Die zumindest eine Ausbuchtung bzw. Erweiterung 44 liegt vorzugsweise auf Höhe der zumindest einen Einbuchtung bzw. der Einkerbung 42, 42` des Sohlenbereichs 28.

Wenn auch im Zusammenhang der Figuren generell von "schematischen" Darstellungen und Ansichten die Rede ist, so ist damit keineswegs gemeint, dass die Figurendarstellungen und deren Beschreibung hinsichtlich der Offenbarung der Erfindung von untergeordneter Bedeutung sein sollen. Der Fachmann ist durchaus in der Lage, aus den schematisch und abstrakt gezeichneten Darstellungen genug an Informationen zu entnehmen, die ihm das Verständnis der Erfindung erleichtern, ohne dass er etwa aus den gezeichneten und möglicherweise nicht exakt maßstabsgerechten Größenverhältnissen des medizinischen und/oder therapeutischen Strumpfes, des Spannmittels oder anderer gezeichneter Elemente in irgendeiner Weise in seinem Verständnis beeinträchtigt wäre. Die Figuren ermöglichen es dem Fachmann als Leser somit, anhand der konkreter erläuterten Umsetzungen des erfindungsgemäßen Verfahrens und der konkreter erläuterten Funktionsweise des erfindungsgemäßen Spannmittels und des durch das erfindungsgemäße Verfahren hergestellten medizinischen und/oder therapeutischen Strumpfes ein besseres Verständnis für den in den Ansprüchen sowie im allgemeinen Teil der Beschreibung allgemeiner und/oder abstrakter formulierten Erfindungsgedanken abzuleiten.

### Bezugszeichenliste

- 10: Medizinischer und/oder therapeutischer Strumpf
- 12: Spannmittel
- 14: Elastisches Zugmittel
- 16: Vorderer Bereich
- 18: Rumpfbereich
- 20: Fersenbereich
- 21: Knöchelbereich
- 22: Öffnung des Strumpfes
- 24: Strumpfmaterial
- 26: Herausragender Abschnitt bzw. überstehender Abschnitt des Spannmittels
- 28: Sohlenbereich
- 30: Strumpfaußenseite
- 32: Strumpfinnenseite
- 34: Aussparung
- 36: Knöchelbereich
- 38: Vorderer Knöchelbereich
- 40: Achillessehnenbereich
- 42: Einbuchtung, Einkerbung
- 42`: Einbuchtung, Einkerbung
- 44: Ausbuchtung, Erweiterung
- 46: Entspannte Stumpfform
- 48: Vorgespannte Strumpfform
- 50: Schaft

## Patentansprüche

1. Verfahren zur Herstellung eines medizinischen und/oder therapeutischen Strumpfes (10), umfassend zumindest die folgenden Schritte:
- Bereitstellen eines Strumpfes mit einem weitgehend geschlossenen vorderen Bereich (16) und einen damit integral verbundenen Rumpfbereich (18) und Fersenbereich (20),
- Vorspannen des gesamten Strumpfes und/oder mindestens eines definierten Abschnittes des Strumpfes durch Beaufschlagen des Strumpfes und/oder des wenigstens einen definierten Abschnittes mit einer durch Einsetzen wenigstens eines Spannmittels (12) in den Strumpf erzeugten Vorspannkraft,
welches Spannmittel (12) zumindest näherungsweise Bereiche und/oder Partien umfasst, die ein menschlicher Fuß aufweist, wobei eine Schablonenkontor des Spannmittels (12) eine Kontur vorgibt, die von einer Fuß- und Beinform eines entspannt stehenden Menschen abweicht,
- definiertes Auflegen und/oder Anordnen und/oder Anheften von mindestens einem elastischen Zugmittel (14) auf das Strumpfmaterial (24) des mittels des Spannmittels (12) vorgespannten Strumpfes und anschließendes Einwirken auf den vorgespannten Strumpf mit dem mindestens einen auf das Strumpfmaterial (24) aufgelegten und/oder angeordneten und/oder angehefteten elastischen Zugmittel (14) mit definierter Temperatur und/oder mit definiertem Druck und/oder unter Verwendung von Haftmittel,
wodurch im Ergebnis ein medizinischer und/oder therapeutischer Strumpf (10) mit mindestens einem applizierten und/oder aufgebrachten elastischen Zugmittel (14) gebildet und/oder hergestellt wird, welches mindestens eine elastische Zugmittel (14) eine Vorspannkraft erzeugt.

2. Verfahren nach Anspruch 1, bei welchem durch die definierte Temperatur das mindestens eine auf das Strumpfmaterial (24) des vorgespannten Strumpfes aufgelegte und/oder angeordnete und/oder angeheftete_elastische Zugmittel (14) zumindest teilweise aufgeschmolzen wird.

3. Verfahren nach Anspruch 1 oder 2, bei welchem bei aufgehobener Temperatureinwirkung das zumindest teilweise aufgeschmolzene mindestens eine elastische Zugmittel (14) erkaltet und/oder erstarrt, wobei sich das mindestens eine elastische Zugmittel (14) mit dem Strumpfmaterial (24) des Strumpfes verbindet.

4. Verfahren nach einem der Ansprüche 1 bis 3, bei welchem zeitlich nach Erkalten und/oder Erstarren des mindestens einen auf das Strumpfmaterial (24) des Strumpfes applizierten und/oder aufgebrachten Zugmittels (14) das zumindest eine Spannmittel (12) aus dem Strumpf entfernt und/oder entnommen wird, so dass der Strumpf in seinen ursprünglichen bzw. entspannten Zustand überführt wird.

5. Verfahren nach einem der Ansprüche 1 bis 4, bei welchem das Einwirken auf den Strumpf mit dem mindestens einen auf das Strumpfmaterial aufgelegten und/oder angeordneten elastischen Zugmittel (14) mit definierter Temperatur und/oder mit definiertem Druck mit einer Zeitdauer von wenigstens fünf Sekunden und höchstens sechzig Sekunden erfolgt.

6. Verfahren nach einem der Ansprüche 1 bis 5, bei welchem das mindestens eine elastische Zugmittel (14) auf einer Seite des Strumpfes oder zu beiden Seiten des vorgespannten Strumpfes zwischen dessen Sohlenbereich (28) und oberhalb der Ferse über den Achillessehnenbereich des vorgespannten Strumpfes angeordnet und/oder auf das Strumpfmaterial (24) des vorgespannten Strumpfes aufgelegt und/oder angeheftet wird und/oder bei welchem das mindestens eine elastische Zugmittel (14) zumindest abschnittsweise horizontal von der Strumpfinnenseite über den Rumpfbereich (18) in Richtung Strumpfaußenseite angeordnet und/oder auf das Strumpfmaterial (24) des vorgespannten Strumpfes aufgelegt und/oder angeheftet wird.

7. Verfahren nach einem der Ansprüche 1 bis 6, bei welchem das mindestens eine elastische Zugmittel (14) zumindest abschnittsweise längsgerichtet und/oder zumindest abschnittsweise schräg verlaufend in den vorderen Strumpfbereich (16) angeordnet und/oder auf das Strumpfmaterial (24) des vorgespannten Strumpfes aufgelegt und/oder angeheftet wird.

8. Verfahren nach einem der Ansprüche 1 bis 7, bei welchem das mindestens eine elastische Zugmittel (14) schräg verlaufend über den oberen Spannbereich bzw. Rumpfbereich (18) des vorgespannten Strumpfes zur Strumpfinnenseite (32) und/oder zur Strumpfaußenseite (30) angeordnet und/oder auf das Strumpfmaterial (24) des vorgespannten Strumpfes aufgelegt und/oder angeheftet wird und/oder bei welchem das mindestens eine elastische Zugmittel (14) unterhalb oder in etwa auf Höhe eines Knöchelbereichs (36) und in ungefähr horizontaler Richtung am vorderen Knöchelbereich (38) des vorgespannten Strumpfes angeordnet und/oder auf das Strumpfmaterial (24) des vorgespannten Strumpfes aufgelegt und/oder angeheftet wird.

9. Verfahren nach Anspruch 8, bei welchem das mindestens eine elastische Zugmittel (14) schräg verlaufend vom Knöchelbereich (38) auf der Strumpfinnenseite (32) über den oberen Spannbereich bzw. Rumpfbereich (18) zur äußeren Sohlenkante angeordnet und/oder auf das Strumpfmaterial (24) des vorgespannten Strumpfes aufgelegt wird, und/oder wobei das mindestens eine elastische Zugmittel (14) von der äußeren Sohlenkante des Strumpfes quer zur Strumpflängsrichtung hinter der Aufnahme für die Zehen entlang der Sohle bis zur Sohleninnenkante und/oder in etwa endend im Bereich der Aufnahme für das Großzehengelenk verlaufend angeordnet und/oder auf das Strumpfmaterial (24) des vorgespannten Strumpfes aufgelegt und/oder angeheftet wird.

10. Verfahren nach einem der Ansprüche 1 bis 9, bei welchem das mindestens eine elastische Zugmittel (14) horizontal verlaufend vom Knöchelbereich (36) der Strumpfinnenseite (32) über den vorderen Knöchelbereich (38) angeordnet und/oder auf das Strumpfmaterial (24) des vorgespannten Strumpfes aufgelegt wird, und/oder welches elastische Zugmittel (14) schräg über den Knöchelbereich (36) der Strumpfaußenseite (30) zur Ferse (20) des vorgespannten Strumpfes führend und/oder von dort wieder nach oben zur Knöchelseite verlaufend angeordnet und/oder auf das Strumpfmaterial (24) des vorgespannten Strumpfes aufgelegt und/oder angeheftet wird.

11. Verfahren nach einem der Ansprüche 1 bis 10, bei welchem das mindestens eine elastische Zugmittel (14) vom Sohlenbereich (20) des Strumpfes schräg verlaufend aufwärts über den Achillessehnenbereich (40) und/oder oberhalb der Ferse (20) des vorgespannten Strumpfes und jeweils beidseitig zurück, mündend vorderhalb der Ferse (20) an den äußeren und inneren Sohlenkanten des Strumpfes angeordnet und/oder auf das Strumpfmaterial (24) des vorgespannten Strumpfes aufgelegt und/oder angeheftet wird.

12. Zumindest ein Spannmittel (12), welches zur Durchführung des Verfahrens und/oder zur Verwendung bei dem Verfahren gemäß einem der Ansprüche 1 bis 11, ausgebildet ist, welches Spannmittel (12) zumindest näherungsweise Bereiche und/oder Partien umfasst, die ein menschlicher Fuß aufweist, wobei das Spannmittel eine Kontur vorgibt, die von der Fuß- und Beinform eines entspannt stehenden Menschen abweicht, wobei das Spannmittel durch eine flache, scheibenartige Schablone gebildet wird,
welches Spannmittel (12) einen Sohlenbereich (28), einen Knöchelbereich (36), einen vorderen Knöchelbereich (38) und einen Achillessehnenbereich (40) aufweist, wobei das Spannmittel (12) im vorderen Knöchelbereich (38) zumindest eine Ausbuchtung oder Erweiterung (44) aufweist.

13. Spannmittel (12) gemäß Anspruch 12, wobei der Achillessehnenbereich (40) und der Sohlenbereich (28) zumindest eine Einbuchtung oder Einkerbung (42) aufweisen, insbesondere eine unterschnittene Form, insbesondere wobei die zumindest eine Einbuchtung oder Einkerbung (42) geschwungen, oder halbkreisförmig oder oval ausgebildet ist.

14. Spannmittel (12) gemäß Anspruch 13, wobei die zumindest eine Ausbuchtung oder Erweiterung (44), in etwa auf Höhe der im Sohlenbereich (28) vorgesehenen zumindest einen Einbuchtung oder Einkerbung (42) angeordnet ist.

15. Spannmittel (12) nach Anspruch 12 oder 13, wobei das Spannmittel (12) mehrteilig ausgebildet ist, insbesondere wobei Verbindungsmittel oder Verbindungselemente vorgesehen sind, über welche die einzelnen Teile des Spannmittels form- und/oder kraftschlüssig zusammensetzbar und/oder zusammenfügbar sind, insbesondere wobei die Verbindungsmittel durch Steck- und/oder Rastverbindungen und/oder um Schraub- und/oder Nietverbindungen sind.

## Claims

1. A method for the manufacture of a medical and/or therapeutic stocking (10) comprising at least the following steps:
- providing a stocking having a largely closed front region (16) and a body region (18) and a heel region (20) integrally connected to said front region,
- pretensioning of the entire stocking and/or at least a defined section of the stocking by applying a pretensioning force to the stocking and/or the at least one defined section of the stocking by inserting at least one stretching means (12) into the stocking, wherein the stretching means (12) at least approximately comprises regions and/or sections, which a human foot exhibits, wherein a stencil outline of the stretching means (12) specifies a contour which deviates from a shape of a foot or leg of a relaxed standing human being,
- defined placing and/or arranging on and/or attaching of at least one elastic tensioning means (14) to the stocking material (24) of the stocking pretensioned by means of the stretching means (12), and subsequent acting on the pretensioned stocking by means of the at least one elastic tensioning means (14) placed and/or arranged on and/or attached to the stocking material (24) at a defined temperature and/or at a defined pressure and/or by using adhesive agents,
wherein, as a result, a medical and/or therapeutic stocking (10) with at least one applied and/or attached elastic tensioning means (14) is formed and/or produced wherein the at least one elastic tensioning means (14) produces a pretensioning force.

2. The method according to claim 1, wherein, by the defined temperature, the at least one tensioning means (14) placed and/or arranged on and/or attached to the stocking material (24) of the pretensioned stocking is at least partly melted.

3. The method according to claim 1 or 2, wherein, with the temperature effect removed, the at least one elastic tensioning means (14) at least partly melted cools and/or solidifies, wherein the at least one elastic tensioning means (14) bonds with the stocking material (24) of the stocking.

4. The method according to any one of claims 1 to 3, wherein temporally after cooling and/or solidifying of the at least one tensioning means (14) applied and/or attached on the stocking material (24) of the stocking, the at least one stretching means (12) is removed and/or taken from the stocking so that the stocking is transferred to its original and/or relaxed state.

5. The method according to any one of claims 1 to 4, wherein acting on the stocking with the at least one elastic tensioning means (14), which is placed and/or arranged on the stocking material, is effected at a defined temperature and/or with a defined pressure for a period of time of at least five seconds and at most sixty seconds.

6. The method according to any one of claims 1 to 5, wherein the at least one elastic tensioning means (14) is arranged on one side of the stocking or on both sides of the pretensioned stocking between the sole region (28) thereof and above the heel over the Achilles tendon region of the pretensioned stocking and/or is placed on and/or attached to the stocking material (24) of the pretensioned stocking and/or wherein the at least one elastic tensioning means (14), at least in sections, is arranged horizontally from the inside of the stocking via the body region (18) in the direction of the outside of the stocking or is placed on and/or attached to the stocking material (24) of the pretensioned stocking.

7. The method according to any one of claims 1 to 6, wherein the at least one elastic tensioning means (14), at least in sections, is arranged lengthwise and/or, at least in sections, extending obliquely into the front region of the stocking (16) and/or is placed on and/or attached to the stocking material (24) of the pretensioned stocking.

8. The method according to any one of claims 1 to 7, wherein the at least one elastic tensioning means (14) is arranged extending obliquely over the upper instep region and/or body region (18) of the pretensioned stocking towards the inside of the stocking (32) and/or towards the outside of the stocking (30) and/or is placed on and/or attached to the stocking material (24) of the pretensioned stocking and/or in which the at least one elastic tensioning means (14) is arranged below or approximately at the level of an ankle region (36) and in an approximately horizontal direction at the front ankle region (38) of the pretensioned stocking and/or is placed on and/or attached to the stocking material (24) of the pretensioned stocking.

9. The method according to claim 8, wherein the at least one elastic tensioning means (14) is arranged extending obliquely from the ankle region (38) on the inside side of the stocking (32) via the upper instep region and/or body region (18) to the outer edge of the sole and/or is placed on the stocking material (24) of the pre-tensioned stocking, and/or wherein the at least one elastic tensioning means (14) is arranged extending from the outer sole edge of the stocking transversely to the longitudinal direction of the stocking behind the receiving means for the toes along the sole to the inner edge of the sole and/or approximately ending in the region of the receiving means for the big toe joint and/or is placed on and/or attached to the stocking material (24) of the pretensioned stocking.

10. The method according to any one of claims 1 to 9, wherein the at least one elastic tensioning means (14) is arranged running horizontally from the ankle region (36) of the inside of the stocking (32) via the front ankle region (38) and/or is placed on the stocking material (24) of the pretensioned stocking, and/or which elastic tensioning means (14) is arranged running obliquely via the ankle region (36) of the outside of the stocking (30) to the heel (20) of the pretensioned stocking and/or running upwards from there again to the ankle side and/or is placed on and/or attached to the stocking material (24) of the pretensioned stocking.

11. The method according to any one of claims 1 to 10, wherein the at least one elastic tensioning means (14), running obliquely upwards from the sole region (20) of the stocking via the Achilles tendon region (40) and/or above the heel (20) of the pretensioned stocking and back on both sides ending in front of the heel (20), is placed on the outer and inner edges of the sole of the stocking and/or is placed on and/or attached to the stocking material (24) of the pretensioned stocking.

12. At least one stretching means (12), which is formed for realization of the method and/or for use in the method according to any one of claims 1 to 11, wherein the stretching means (12) at least approximately comprises regions and/or sections, which a human foot exhibits, wherein the stretching means specifies a contour which deviates from the shape of a foot or a leg of a relaxed standing human being, wherein the stretching means is formed by a flat, disk-like stencil,
wherein the stretching means (12) comprises a sole region (28), an ankle region (36), a front ankle region (38) and an Achilles tendon region (40), wherein the stretching means (12) comprises at least a bulge or an extension (44) in the front ankle region (38).

13. The stretching means (12) according to claim 12, wherein the Achilles tendon region (40) and the sole region (28) comprise at least one indentation or notch (42), in particular an undercut shape, in particular wherein the at least one indentation or notch (42) has a curved, a semicircular or oval shape.

14. The stretching means (12) according to claim 13, wherein the at least one bulge or extension (44) is arranged approximately at the level of the at least one indentation or notch (42) provided in the sole region (28).

15. The stretching means (12) according to claim 12 or 13, wherein the stretching means (12) is formed in several parts, in particular wherein connecting means or connecting elements are provided by means of which the individual parts of the stretching means can be assembled and/or joined by positive or non positive locking, in particular wherein the connecting means are plug-in and/or latching connections and/or screw and/or rivet connections.

## Revendications

1. Procédé pour la fabrication d'un bas médical et/ou thérapeutique (10) comportant les étapes suivantes:
- fourniture d'un bas présentant une partie avant (16) dans une large mesure fermée, formant une seule pièce avec une partie de corps (18) et une partie de talon (20),
- précontrainte du bas entier et/ou d'au moins une section définie du bas par soumission du bas et/ou de ladite au moins une section définie à une force de précontrainte par utilisation d'au moins un moyen de tension (12) dans le bas, dans lequel le moyen de tension (12) comporte au moins approximativement des zones et/ou des parties que présente un pied humain, dans lequel un contour du stencil du moyen de tension (12) définit un contour qui s'écarte de la forme du pied ou de la jambe d'une personne debout et détendue,
- placement et/ou agencement et/ou fixation de manière définie de l'au moins un moyen de traction élastique (14) sur le matériau (24) du bas précontraint par l'intermédiaire du moyen de tension (12) et ensuite action sur le bas précontraint avec l'au moins un moyen de traction élastique (14) placé et/ou agencé et/ou fixé sur le matériau du bas (24) à une température définie et/ou à une pression définie et/ou à l'aide de moyens d'adhérence,
de manière à former et/ou fabriquer de ce fait un bas (10) médical et/ou thérapeutique présentant au moins un moyen de traction (14) élastique appliqué et/ou disposé sur celui-ci, dans lequel l'au moins un moyen de traction élastique (14) produit une force de précontrainte.

2. Procédé selon la revendication 1, dans lequel, par la température définie, l'au moins un moyen de traction élastique (14) placé et/ou agencé et/ou fixé sur le matériau (24) du bas précontraint est fondu au moins partiellement.

3. Procédé selon la revendication 1 ou 2, dans lequel, lorsque l'effet de la température est supprimé, l'au moins un moyen de traction élastique (14) au moins partiellement fondu se refroidit et/ou se solidifie, dans lequel l'au moins un moyen de traction élastique (14) se lie au matériau (24) du bas.

4. Procédé selon l'une des revendications 1 à 3, dans lequel, chronologiquement après le refroidissement et/ou la solidification de l'au moins un moyen de traction (14) appliqué et/ou disposé sur le matériau (24) du bas, l'au moins un moyen de tension (12) est retiré et/ou enlevé du bas, de sorte que le bas est transféré dans son état initial ou détendu.

5. Procédé selon l'une quelconque des revendications 1 à 4, dans lequel l'action sur le bas avec ledit au moins un moyen de traction élastique (14) placé et/ou agencé sur le matériau du bas est effectuée à une température définie et/ou à une pression définie avec une durée de temps d'au moins cinq secondes et d'au plus soixante secondes.

6. Procédé selon l'une des revendications 1 à 5, dans lequel l'au moins un moyen de traction élastique (14) est agencé d'un côté du bas ou des deux côtés du bas précontraint entre sa zone de semelle (28) et au-dessus du talon sur la zone du tendon d'Achille du bas précontrainte et/ou est placé et/ou fixé sur le matériau (24) du bas précontrainte, et/ou dans lequel l'au moins un moyen de traction élastique (14) est agencé au moins par sections horizontalement depuis le côté intérieur du bas en passant par la partie de corps (18) en direction du côté extérieur du bas et/ou est placé et/ou fixé sur le matériau (24) du bas précontraint.

7. Procédé selon l'une des revendications 1 à 6, dans lequel l'au moins un moyen de traction élastique (14) est agencé au moins par sections longitudinalement et/ou au moins par sections s'étendant en oblique dans la partie avant du bas (16) et/ou est placé et/ou fixé sur le matériau (24) du bas précontraint.

8. Procédé selon l'une quelconque des revendications 1 à 7, dans lequel l'au moins un moyen de traction élastique (14) en s'étendant en oblique est agencé sur la zone supérieure du cou-de-pied et/ou sur la partie de corps (18) du bas précontraint vers le côté intérieur (32) du bas et/ou vers le côté extérieur (30) du bas et/ou est placé et/ou fixé sur le matériau (24) du bas précontraint et/ou dans lequel l'au moins un moyen de traction élastique est placé en dessous ou à peu près au niveau d'une zone de la cheville (36) et dans une direction approximativement horizontale sur la zone avant de la cheville (38) du bas précontraint et/ou est placé et/ou fixé sur le matériau du bas (24) précontraint.

9. Procédé selon la revendication 8, dans lequel l'au moins un moyen de traction élastique (14) est placé s'étendant en oblique depuis la zone de la cheville (38) sur le côté intérieur du bas (32) en passant par la zone supérieure du cou-de-pied et/ou par la partie de corps (18) jusqu'au bord extérieur de la semelle et/ou est placé sur le matériau du bas (24) précontraint, et/ou dans lequel l'au moins un moyen de traction élastique (14) est agencé en s'étendant depuis le bord extérieur de la semelle du bas transversalement à la direction longitudinale du bas derrière le logement pour les orteils, le long de la semelle jusqu'au bord intérieur de la semelle et/ou se terminant à peu près dans la zone du logement pour l'articulation du gros orteil, et/ou est placé et/ou fixé sur le matériau (24) du bas précontraint.

10. Procédé selon l'une quelconque des revendications 1 à 9, dans lequel l'au moins un moyen de traction élastique (14) est placé en s'étendant horizontalement depuis la zone de cheville (36) du côté intérieur du bas (32) en passant par la zone de cheville avant (38) et/ou est placé sur le matériau (24) du bas précontraint, et/ou dans lequel le moyen de traction élastique (14) est placé en s'étendant en oblique sur la zone de la cheville (36) du côté extérieur (30) du bas vers le talon (20) du bas précontraint et/ou en s'étendant de là à nouveau vers le haut vers le côté de la cheville et/ou est placé et/ou fixé sur le matériau (24) du bas précontraint.

11. Procédé selon l'une quelconque des revendications 1 à 10, dans lequel l'au moins un moyen de traction élastique (14) est agencé s'étendant obliquement depuis la zone de semelle (20) du bas vers le haut en passant par la zone du tendon d'Achille (40) et/ou au-dessus du talon (20) du bas précontraint et respectivement des deux côtés en retour, débouchant à l'avant du talon (20) sur les bords extérieurs et intérieurs du bas et/ou est placé et/ou fixé sur le matériau (24) du bas précontraint.

12. Au moins un moyen de tension (12), qui est configuré pour réaliser le procédé et/ou pour l'utilisation dans le procédé selon l'une quelconque des revendications 1 à 11, dans lequel le moyen de tension (12) comporte au moins approximativement des zones et/ou des parties que présente un pied humain, dans lequel le moyen de tension (12) définit un contour qui s'écarte de la forme du pied et de la jambe d'une personne debout et détendue, dans lequel le moyen de tension (12) est formé par un stencil plat en forme de disque,
dans lequel le moyen de tension comporte une zone de semelle (28), une zone de la cheville (36) une zone avant de cheville (36) et une zone du tendon d'Achille (40), dans lequel le moyen de tension (12), dans la zone avant de cheville (38), comporte au moins un cambrage ou une extension (44).

13. Moyen de tension (12) selon la revendication 12, dans lequel la zone du tendon d'Achille (40) et la zone de semelle (28) comportent au moins une échancrure ou une rainure (42), surtout une forme écrénée, surtout dans lequel l'au moins une échancrure ou une rainure (42) est en forme courbée ou semi-circulaire ou ovale.

14. Moyen de tension (12) selon la revendication 13, dans lequel l'au moins un cambrage ou l'extension (44) est agencé à peu près à la hauteur de l'au moins une échancrure ou une rainure (42) prévue dans la zone de la semelle (28).

15. Moyen de tension (12) selon la revendication 12 ou 13, dans lequel le moyen de tension (12) est formé en plusieurs parties, surtout dans lequel des moyens de liaison ou des éléments de liaison sont prévus par lesquels les différentes parties du moyen de tension peuvent être assemblées et/ou jointes à engagement positif ou par adhérence, surtout dans lequel les moyens de liaison sont des connecteurs et/ou des raccords par encliquetage et/ou des raccords à vis et/ou des raccords par rivetage.
